Europäisches Patentamt

European Patent Office

Office européen des brevets

(11) Publication number: **0 133 382**
**A1**

(12) # EUROPEAN PATENT APPLICATION

(21) Application number: **84305379.4**

(22) Date of filing: **07.08.84**

(51) Int. Cl.⁴: **A 61 B 5/05, H 01 F 17/00,**
**G 01 N 24/08**

(30) Priority: **08.08.83 GB 8321308**

(43) Date of publication of application: **20.02.85**
**Bulletin 85/8**

(84) Designated Contracting States: **DE FR GB IT NL SE**

(71) Applicant: **M & D TECHNOLOGY LIMITED,**
**18 Bon-Accord Crescent, Aberdeen AB9 1XL (GB)**

(72) Inventor: **Mallard, John Rowland, 121 Anderson Drive,**
**Aberdeen AB2 6BG Scotland (GB)**
Inventor: **Smith, David Balfour, East Neuk Netherley,**
**Stonehaven (GB)**
Inventor: **Eastwood, Linda Mary, 36 Thomson Street,**
**Aberdeen, AB2 4QP Scotland (GB)**
Inventor: **Redpath, Thomas William Tennant, 62 Watson**
**Street, Aberdeen AB2 4SU Scotland (GB)**
Inventor: **Hutchison, James MacDonald Strachan,**
**29 Lawsondale Drive Westhill, Aberdeen, AB3 6TU**
**Scotland (GB)**
Inventor: **Selbie, Robert Dass, 218 Deeside Gardens,**
**Aberdeen, AB1 7PS Scotland (GB)**
Inventor: **Johnson, Glyn, 17 St. James Drive,**
**Wandsworth Common London, SW17 7RN (GB)**

(74) Representative: **Skone James, Robert Edmund et al,**
**GILL JENNINGS & EVERY 53-64 Chancery Lane, London**
**WC2A 1HN (GB)**

(54) **Radiofrequency coil.**

(57)    A radiofrequency coil (1) is described which comprises a pair of electrically conductive loops (4, 5) connected in series. The loops (4, 5) are arranged substantially symmetrically about a plane (6) whereby in use a radiofrequency magnetic field induced between the loops (4, 5) passes through the plane (6) substantially at right angles to the plane. The coil (1) is particularly applicable for use in NMR imaging apparatus.

M & D TECHNOLOGY LIMITED                    52/2090/02

RADIOFREQUENCY COIL

The invention relates to a coil for generating a radiofrequency (R.F.) magnetic field in response to the flow of an R.F. current through the coil. The invention is particularly related to the use of such a coil in nuclear magnetic resonance (NMR) imaging apparatus.

Nuclear magnetic resonance relies on the fact that when certain nuclei are exposed to a uniform static magnetic field they will precess around the axis of the applied magnetic field with a frequency proportional to the strength of the field. This frequency is known as the Larmor frequency ($W_0$) and is given by the formula

$$W_0 = \gamma H_0$$

where $\gamma$ is the gyromagnetic ratio of the particular nucleus and $H_0$ is the strength of the magnetic field. By applying a static field which varies in strength along a particular direction, nuclei at each position in that direction precess at different frequencies. Thus, by applying a radio frequency magnetic field of sufficient strength at the same time as supplying a gradient magnetic field across the object, only those nuclei with spins precessing with the frequency or frequencies of the pulse can be made to rotate through 90° or 180° and are thus isolated from the remainder of the nuclei.

The R.F. magnetic field is generated and received using coils such as solenoids with their axes arranged in appropriate directions. One such coil is described in a paper entitled "Design of a Radiofrequency Coil suitable for NMR Imaging of Heads" published in Phys. Med. Biol. 1982 Vol. 27, No. 3, pages 443-447. This R.F. coil has a cyclindrical form and is used for improving the signal-to-noise ratio of NMR head images.

These known coils are not suitable for use in imaging a patient's neck as well as his head.

In accordance with the present invention, a radiofrequency coil comprises a pair of electrically conductive loops connected in series, the loops being arranged substantially symmetrically about a plane whereby in use a radiofrequency magnetic field induced between the loops passes through the plane substantially at right angles to the plane.

In one example, each loop comprises a first portion extending in a first plane and a second portion extending in a second plane, the first and second planes being arranged substantially at right angles to one another. This arrangement is particularly suitable for improving the NMR imaging of a head and neck since a patient's shoulders can be easily received between the first and second planes enabling the magnetic field to be substantially uniform through the patient's head and neck and leading to a better signal-to-noise ratio than can be obtained with a larger whole body coil because of the improved coil filling factor.

In practice, the loops are integrally connected, the coil having a pair of ends defined by opposed ends of respective loops. The coil is made from a single length of wire the free ends of which may be conveniently close to the plane of symmetry. This will minimise the length and resistance of capacitor leads when a tuning capacitor is connected across the ends and it has the further advantage that stray magnetic fields due to currents in the leads to the ends will be minimised.

A further advantage of this coil is that it can be easily positioned about a former. Thus in one example, the coil is formed as a figure of eight and is positioned around a former with the coil loops laid flat on or wrapped around the former. This considerably simplifies the construction of the loops since the coil can be positioned on the former without the necessity of lifting parts of the coil to pass other parts underneath.

In the past, single R.F. coils have been used in NMR imaging apparatus both to transmit and receive an R.F. magnetic field, a suitable switch being provided in the circuitry to which the coil is connected so that the coil is connected alternately to a transmitter and a receiver.

A coil in accordance with the present invention could be used in this manner but is preferably used as a receiving coil only when in the form of a head and neck coil.

We have recognised, however, that this new coil could also be used in conventional whole body NMR apparatus instead of the normal solenoid for generating or receiving an R.F. magnetic field. Preferably, a radiofrequency coil in accordance with the invention is connected to a transmitter and is positioned to generate a R.F. magnetic field along a first direction, a solenoid connected to a receiver being positioned with its axis extending in a second direction transverse to the first direction. This arrangement avoids the need for a switching unit and the transverse arrangement of the first and second directions reduces the mutual inductance between the coil and the solenoid. Alternatively, the R.F. coil could be connected to the receiver and the solenoid to the transmitter. Conveniently, the solenoid is positioned centrally within the coil. This reduces the space required for the coils.

The arrangement of separate transmitting and receiving coils can be used both for whole body NMR imaging and head and neck NMR imaging as previously described.

An example of a radiofrequency coil in accordance with the present invention will now be described with reference to the accompanying drawings, in which:-

Figure 1 illustrates the coil mounted on a former;

Figure 2 diagrammatically illustrates the two loops disconnected from each other:

Figure 3 illustrates the coil when laid flat; and,

Figure 4 i is an exploded view illustrating NMR imaging apparatus incorporating an enlarged coil.

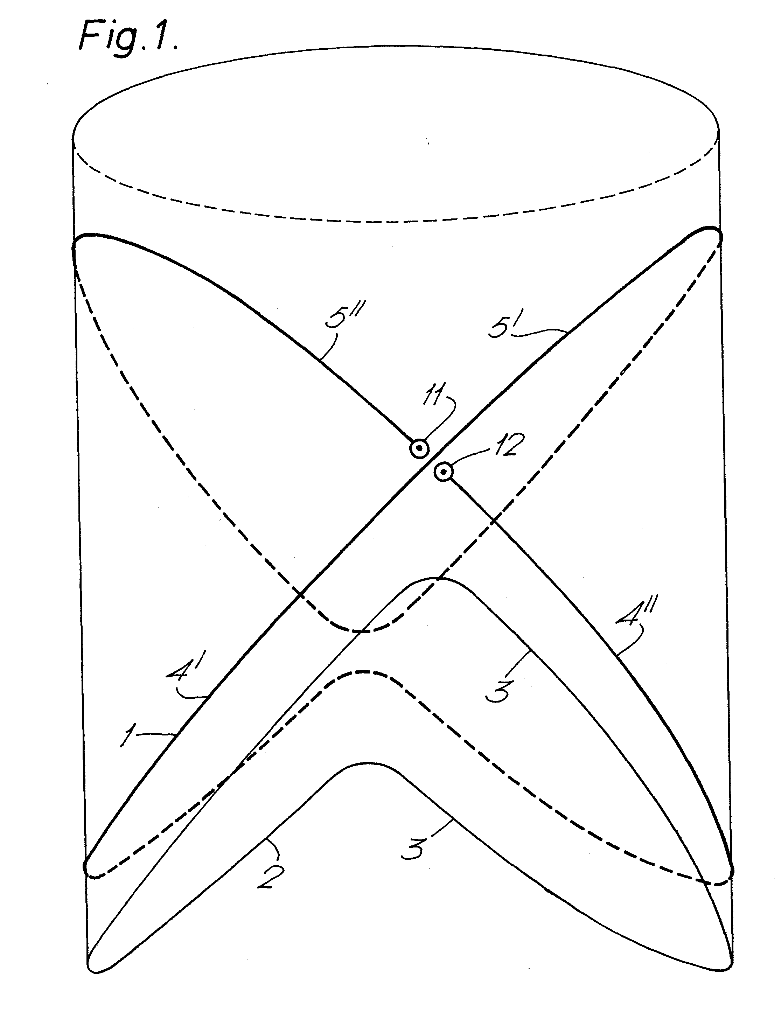

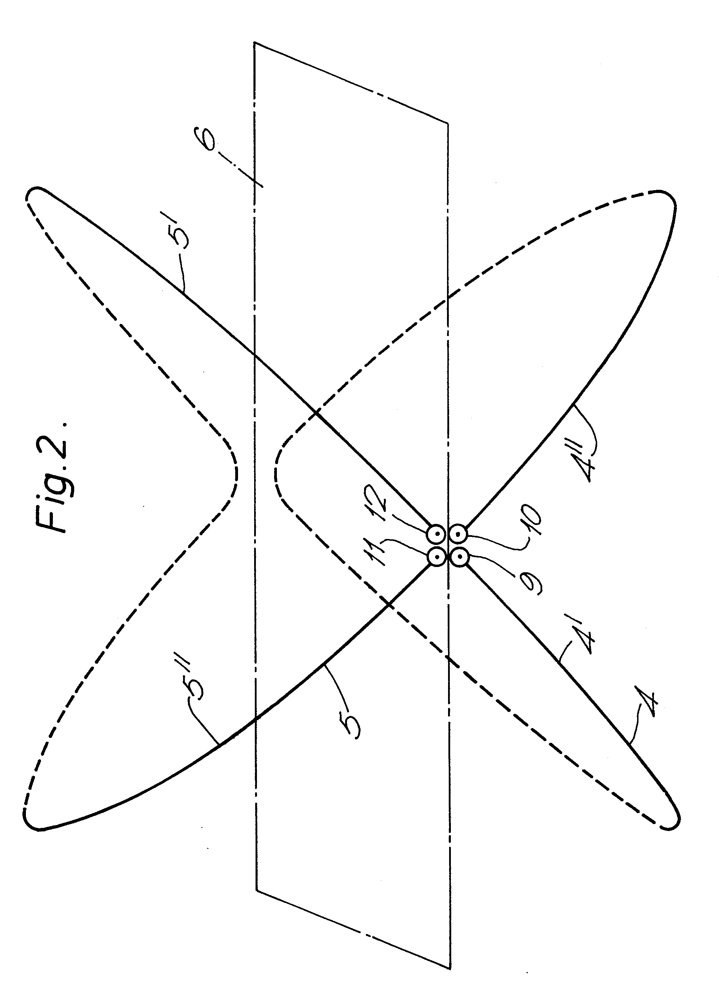

Figure 1 illustrates a R.F. coil 1 mounted on a tubular former 2 having a pair of cut-outs 3. The effective form of the coil 1 can be seen more clearly in Figure 2 where the coil is broken down into its pair of loops 4, 5. In Figure 2, the former 2 has been omitted for clarity. As can be seen, the loop 4 comprises two sections 4', 4" while the loop 5 comprises two sections 5', 5". The section 4' is coplanar with the section 5' and the section 4" is coplanar with the section 5". Also, the two loops 4, 5 are arranged symmetrically with one another about a plane 6 shown in phantom. In Figure 2, each end of each loop 4, 5 is illustrated, the ends of the loop 4 being indicated by references 9, 10 and the ends of the loop 5 being indicated by the references 11, 12. In practice, the ends 9, 12 do not exist and the loop 4' is integral with the loop 5' as is shown in Figure 1. In use, when a R.F. current is applied to the ends 10, 11 a R.F. magnetic field is generated between the loops 4, 5 and due to the symmetrical arrangement of the loops this magnetic field will pass through the plane of symmetry 6 at right angles to the plane and fill the volume defined by the coil 1.

Thus, when the coil 1 is used as a head and neck coil in NMR imaging it will be positioned on the former 2 as shown in Figure 1 and the former 2 will be placed over a patient's head so that the cut-outs 3 receive the patient's shoulders and the lower ends of the coil 1 formed by the sections 4', 4" of the loop 4 will extend in front and behind the patient's neck respectively. The ends 10, 12 of the coil 1 form a pair of terminals which are connected to conventional apparatus for receiving or generating a R.F. current.

Typically, the conducting material from which the loop 1 is formed will be as thick as possible to minimise R.F. resistance.

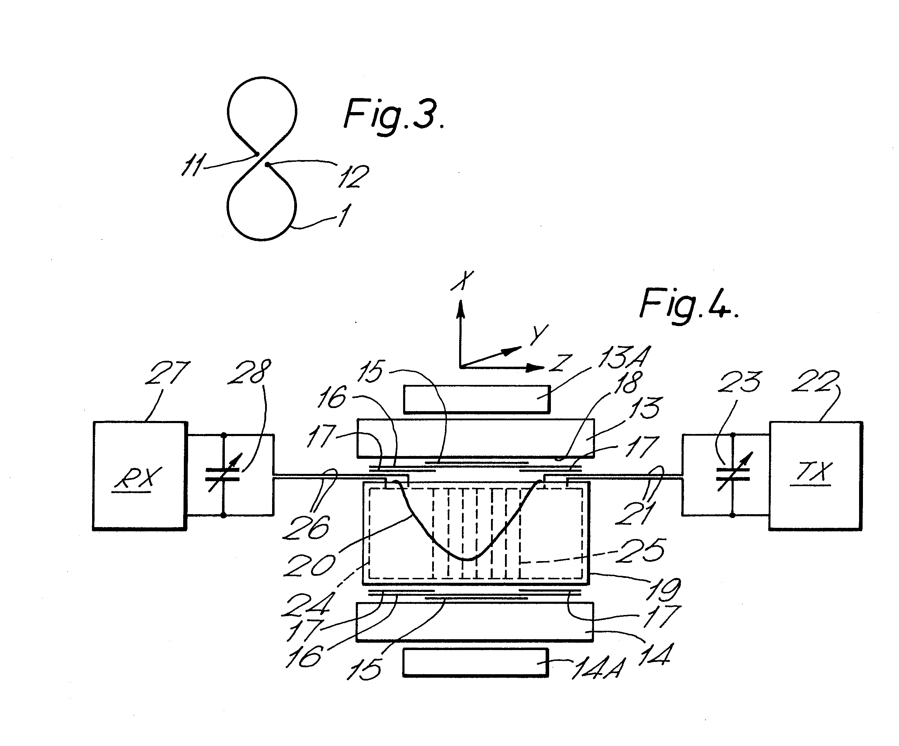

Figure 3 illustrates the loop 1 before mounting on the former 2. It will be seen that the loop 1 is shaped in the form of a figure-of-eight and this is particularly useful since it can then simply be laid on and wrapped around the former 2 after it has been formed into the shape shown in Figure 3. In particular, it should be noted that there is no cross-over of the conducting material forming the loop. This considerably simplifies the mounting of the loop 1 on the former 2.

When the loop 1 is used in connection with NMR head and neck imaging it can conveniently be connected to a conventional R.F. current receiver and used in conjunction with NMR imaging apparatus as for example the imaging apparatus currently made and sold by M & D Technology Limited. Alternatively, it could be used both to generate and detect a R.F. magnetic field in which case the terminals 11, 12 will be connected via a suitable switch alternately to a receiver and a transmitter. The switch will be controlled by a suitable computer in a manner well known in the art.

Another application of the loop 1 shown in Figure 1 is as a R.F. magnetic field generator in whole-body NMR imaging apparatus. Such apparatus is indicated schematically in Figure 4 and may be the imaging apparatus made and sold by M & D Technology Limited. The apparatus comprises a magnet having coils 13, 13A, 14, 14A for generating a static magnetic field in the X direction typically having a magnitude of 0.08T. Three sets of gradient coils 15, 16, 17 are mounted coaxially within a bore 18 defined by the coils 13, 14. These gradient coils 15-17 are arranged to cause the magnetic field within the bore 18 to vary in a controlled manner across the space defined by the bore 18 so that nuclei at different positions within the space will precess. The coils 15-17 are connected to conventional current sources (not shown).

A former 19 is mounted coaxially within the bore 18 and a R.F. coil 20 having a form similar to that shown in Figure 1 but a larger diameter is mounted around the former 19. The coil 20 is connected via leads 21 to a R.F.current transmitter 22 and a tuning capacitor 23. When the transmitter 22 is actuated a R.F. current is caused to flow through the loop 20 which in turn causes a R.F. magnetic field to be generated in a direction parallel with the Y axis. As has previously been explained in connection with the examples shown in Figures 1 and 2 this magnetic field will pass through the plane of symmetry (not shown) of the loop 20 at right angles to the plane.

Within the cylindrical former 19 is positioned another cylindrical former 24 around which is positioned a solenoid 25.

The solenoid 25 is connected via leads 26 to a R.F. current receiver 27 and a tuning capacitor 28.

The shape of the formers 19, 24 can be circular as mentioned but other cross-sections could be used such as eliptical.

In practice the coils 13, 14 will be closer together than shown so that parts of the formers 19, 24 will be within the coils. In view of this, the available space for a patient will be defined in the X direction by the spacing of the coils 13, 14 rather than the diameter of the former 24.

0133382

In use, a patient is positioned within the former 24 along the Z axis and a static magnetic field is set up between the coils 13, 13A, 14, 14A. The transmitter 22 in conjuction with the tuning capacitor 23 is then caused to pass a R.F. current through the coil 20 to generate a R.F. magnetic field within the former 24. The gradient coils 15-17 are then actuated in accordance with a predetermined programme and the resultant R.F. magnetic field causes a corresponding current to flow through the windings of the solenoid 25. This R.F. current is detected by the receiver 27. An example of such a process is illustrated in British Patent Specification No. 2,125,563. As described in that specification, the R.F. magnetic field causes the spins of appropriate nuclei to flip through 90° or 180°.

Operation of the transmitter 22 and the coils 15-17, and processing of the output of the receiver 27 is carried out by a computer (not shown) in a manner well known to those skilled in the art.

0133382

## CLAIMS

1. A radiofrequency coil (1) comprising a pair of electrically conductive loops (4,5) connected in series, the loops (4,5) being arranged substantially symmetrically about a plane (6) whereby in use a radiofrequency magnetic field induced between the loops (4,5) passes through the plane (6) substantially at right angles to the plane.

2. A coil according to claim 1, wherein each loop comprises a first portion (4',5') extending in a first plane and a second portion (4",5') extending in a second plane, the first and second planes being arranged substantially at right angles to one another.

3. A coil according to claim 1 or claim 2, wherein the loops are integrally connected, the coil having a pair of ends (11,12) defined by opposed ends of respective loops (4,5).

4. A coil according to claim 3, wherein the ends (11,12) of the coil are positioned close to the plane of symmetry (6).

5. A coil according to any of the preceding claims, wherein the coil is in the form of a figure-of-eight.

6. A coil according to any of the preceding claims mounted on a former (2).

7. A head and neck coil (1) according to claim 6.

8. NMR imaging apparatus incorporating a radiofrequency coil in accordance with any of the preceding claims.

9. NMR imaging apparatus according to claim 8, wherein the coil (20) is connected to a R.F. current generator (22), the imaging apparatus further comprising a solenoid (25) connected to a radiofrequency current detector (27) for detecting a R.F. magnetic field in the apparatus.

10. NMR imaging apparatus according to claim 9, wherein the coil (20) is positioned to generate a R.F. magnetic field along a first direction (X), the solenoid (25)

9          0133382

being positioned with its axis extending in a second
direction (Y) transverse to the first direction.

**Fig.1.**

Fig.2.

2/3

0133382

0133382

Fig.3.

Fig.4.

| | **DOCUMENTS CONSIDERED TO BE RELEVANT** | | EP 84305379.4 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | GB - A - 1 596 160 (NATIONAL RESEARCH DEVELOPMENT)<br><br>* Page 8, lines 50-59; page 10, lines 51-61; fig. 12,19 *<br><br>-- | 1-6,8, 9 | A 61 B 5/05<br><br>H 01 F 17/00<br><br>G 01 N 24/08 |
| X,P | EP - A2 - 0 091 008 (GENERAL ELECTRIC) (12-10-1983)<br><br>* Abstract; page 23, lines 14-26; fig. 1b,8c *<br><br>-- | 1-6,8, 9 | |
| X,P | EP - A1 - 0 086 972 (GENERAL ELECTRIC) (31-08-1983)<br><br>* Abstract; page 20, lines 14-25; fig. 11b,11c *<br><br>-- | 1-6,8, 9 | |
| X | DE - A1 - 3 202 368 (NATIONAL RESEARCH DEVELOPMENT)<br><br>* Page 20, line 21 - page 22, line 18; fig. 2-3c *<br><br>-- | 1,3,4, 6,8,9 | **TECHNICAL FIELDS SEARCHED (Int. Cl.4)**<br><br>A 61 B<br><br>H 01 F<br><br>G 01 N |
| X | DE - A1 - 2 604 301 (NIHON DENSHI K.K.)<br><br>* Page 6, line 9 - page 7, line 6; fig. 1,5 *<br><br>-- | 1,3,4, 8,9 | |

The present search report has been drawn up for all claims

| Place of search | Date of completion of the search | Examiner |
|---|---|---|
| VIENNA | 08-11-1984 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

0133382

| | **DOCUMENTS CONSIDERED TO BE RELEVANT** | | EP 84305379.4 |
|---|---|---|---|
| Category | Citation of document with indication, where appropriate, of relevant passages | Relevant to claim | CLASSIFICATION OF THE APPLICATION (Int. Cl.4) |
| X | A. BOEKHORST, J. STOLK "Ablenk-technik in Fernseh-Empfängern", 1961<br><br>PHILIPS TECHNISCHE BIBLIOTHEK, CENTREX VERLAG, Eindhoven pages 70-75, 80-87<br><br>    * Fig. 5.4.1.2., 5.5.1.1., 5.5.7.3. * | 1 | |
| | | | **TECHNICAL FIELDS SEARCHED (Int. Cl.4)** |

The present search report has been drawn up for all claims

| Place of search | Date of completion of the search | Examiner |
|---|---|---|
| VIENNA | 08-11-1984 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82